# EUROPEAN PATENT APPLICATION

(11) **EP 3 150 196 A1**
(43) Date of publication of application: **05.04.2017**
(21) Application number: 16191594.7
(22) Date of filing: 29.09.2016
(51) Int. Cl.: A61K 9/127, A61K 31/7048

(54) **PROCESS FOR THE PREPARATION OF UNILAMELLAR LIPOSOMAL COMPOSITION**

(30) Priority: 30.09.2015 IN 3710MU2015
(71) Applicant: Sun Pharma Advanced Research Company Ltd, 400093 Mumbai (IN)
(72) Inventor: NAIK, Sachin, 390020 Baroda (IN); SEJU, Ujjawal, 390020 Baroda (IN); BHOWMICK, Subhas, 390020 Baroda (IN)
(74) Representative: O'Neill, Michelle

(57) **Abstract**

The present invention relates to a process for the preparation of unilamellar liposomal composition of a polyene antibiotic drug. The present invention further relates to liposomal composition of a polyene antibiotic drug that is stable.

## Description

### FIELD OF THE INVENTION

The present invention relates to a process for the preparation of unilamellar liposomal composition of a polyene antibiotic drug that are stable.

### BACKGROUND OF THE INVENTION

Polyene antibiotics such as amphotericin B and nystatin are indicated for the treatment of life threatening systemic fungal infections such as candidosis and aspergillosis. The polyene antibiotics are amphiphilic in nature and are practically insoluble in aqueous medium as well as most of organic solvents, which makes it difficult to formulate a stable dosage form of these drugs. Few lipid-based amphotericin B formulations have been developed and marketed.

One such formulation is AmBisome^{®}, which is a unilamellar liposomal preparation developed by Vestar Inc. U.S.A. AmBisome^{®} is approved by USFDA. The United States patent number 5,965,156 (herein after referred to as the '156 patent) describe a process of formation of a unilamellar liposomal composition containing a polyene antibiotic. However the patent neither describes the formation of drug related impurities nor describes the impurities formed by degradation of the lipids or the phospholipids.

When the prior art process as disclosed in the '156 patent was followed to prepare the liposomes of amphotericin B, the inventors found that the liposome resulted with higher and unacceptable level of lipid impurities and the liposomes formed had vesicles with distorted morphology, i.e. were uneven in size and shape. The formation of degradants of the lipids is a known problem. It is known that due to chemical hydrolysis of liposomal phospholipids, the organization of the lipid assembly can change which can convert the liposome from its desirable lamellar structure into a micellar system. It has been studied that the presence of unacceptable levels of hydrolysis products of phospholipids is the indicator of unstable liposome preparation. (See N.J. Zuidam et al. / Biochimica et Biophysica Acta 1240 (1995") 101-110*).*

While arriving at a stable liposome composition, the inventors tried enumerable modifications in the processes and arrived at a process that yielded a liposome composition which is stable, such that the liposome composition have reduced levels of lipid degradation impurities. Also, the resulting liposome vesicles have uniform, well defined, spherical shape and size. Additionally, the drug related impurities are well within acceptable limits.

### SUMMARY OF THE INVENTION

The present invention provides a process for the preparation of an unilamellar liposomal composition containing a polyene antibiotic, said process comprising forming a soluble complex between the polyene antibiotic and a phospholipid in an acidified organic solvent and maintaining said soluble complex at a pH of 4.5 or less, removing the organic solvent to form a dried complex and hydrating the dried complex with an aqueous buffer solution and forming a dispersion of unilamellar liposomes containing the polyene antibiotic from the hydrated mixture, subjecting the dispersion to filtration sterilization,
the process comprising additional step of adding a buffer having a pH of 3 to 6 to the dispersion of unilamellar liposomes before the step of filtration sterilization.

Particularly, the present invention provides a process for the preparation of an unilamellar liposomal composition containing a polyene antibiotic, said process comprising forming a soluble complex between the polyene antibiotic and a phospholipid selected from phosphatidylglycerol or phosphatidylcholine or mixtures thereof, in an acidified organic solvent and maintaining said soluble complex at a pH of 4.5 or less, removing the organic solvent to form a dried complex and hydrating the dried complex with an aqueous buffer solution and forming a dispersion of unilamellar liposomes containing the polyene antibiotic from the hydrated mixture, subjecting the dispersion to filtration sterilization,
the process comprising an additional step of adding a buffer having a pH of 3 to 6 to the dispersion of unilamellar liposomes before the step of filtration sterilization,
wherein the lyso phosphatidylglycerol is present in the liposomal composition at a concentration ranging from 0.1 % to 4.0 % by weight of phosphatidylglycerol and lyso phosphatidylcholine is present in the liposomal composition at a concentration ranging from 0.1 % to 3.0 % by weight of phosphatidylcholine.

In one aspect, the present invention provides a liposomal composition of a polyene antibiotic drug prepared by the process as described herein.

Alternatively, the present invention can be described as providing a liposomal composition comprising:
amphotericin B in an amount ranging from 1 % to 8 % by weight,
phosphatidylglycerol in an amount ranging from 2 % to 10 % by weight, phosphatidylcholine in an amount ranging from 10 % to 20 % by weight, and
cholesterol in an amount ranging from 2 % to 8 % by weight of final liposomal composition, wherein the lyso phosphatidylcholine impurity is between 0.1 to 3.0 % by weight of the phosphatidylcholine and lyso phosphatidylglycerol impurity is between 0.1 to 4.0 % by weight of the phosphatidylglycerol, when tested at any time during the shelf life of the product.

Particularly, the present invention can be described as providing a liposomal composition comprising
Amphotericin B in an amount ranging from 1 % to 8 % by weight,
phosphatidylglycerol in an amount ranging from 2 % to 10 % by weight,
phosphatidylcholine in an amount ranging from 10 % to 20 % by weight,
cholesterol in an amount ranging from 2 % to 8 % by weight of final liposomal composition, wherein the liposomal composition is obtained by a process comprising forming a soluble complex between the polyene antibiotic and a phospholipid in an acidified organic solvent and maintaining said soluble complex at a pH of 4.5 or less, removing the organic solvent to form a dried complex and hydrating the dried complex with an aqueous buffer solution and forming a dispersion of unilamellar liposomes containing the polyene antibiotic from the hydrated mixture, subjecting the dispersion to filtration sterilization, the process comprising additional step of adding a buffer having a pH of 3 to 6 to the dispersion of unilamellar liposomes before the step of filtration sterilization,
wherein the lyso phosphatidylcholine impurity is between 0.1 to 3.0 % by weight of the phosphatidylcholine and lyso phosphatidylglycerol impurity is between 0.1 to 4.0 % by weight of the phosphatidylglycerol.

Particularly, the present invention can be described to provide a liposomal composition comprising
amphotericin B in an amount of about 3.5 % by weight,
phosphatidyl glycerol in an amount of about 6.0 % by weight,
phosphatidylcholine in an amount of about 15.0 % by weight, and
cholesterol in an amount of about 3.5 % by weight, of final liposomal composition,
wherein the liposomal composition is obtained by a process comprising forming a soluble complex between the polyene antibiotic and a phospholipid in an acidified organic solvent and maintaining said soluble complex at a pH of 4.5 or less, removing the organic solvent to form a dried complex and hydrating the dried complex with an aqueous buffer solution and forming a dispersion of unilamellar liposomes containing the polyene antibiotic from the hydrated mixture, subjecting the dispersion to filtration sterilization, the process further comprising an additional step of adding a buffer having a pH of 3 to 6 to the dispersion of unilamellar liposomes before the step of filtration sterilization,
wherein the lyso phosphatidylcholine impurity is about 1.83 % by weight of the phosphatidylcholine and lyso phosphatidyl glycerol impurity is about 2.98 % by weight of the phosphatidylglycerol.

### DESCRIPTION AND DISCUSSION OF THE FIGURES

Figure 1 represents the image of liposomes prepared as per Example 1. The image is recorded by cryo-transmission electron microscopy. The image reveals well-defined, structured and spherical morphology of the lamellar liposome.
Figure 2 represents the image of liposomes prepared as per comparative Example 1. The image is recorded by cryo-transmission electron microscopy. The image reveals vesicles have a distorted morphology with uneven size and shape.
Figure 3 represents the image of liposomes prepared as per comparative Example 2. The image is recorded by cryo-transmission electron microscopy. The image reveals vesicles have a distorted morphology with uneven size and shape.

### DETAILED DESCRIPTION OF THE INVENTION

The term 'Liposomes', as used herein refers to artificially prepared spherical vesicles composed of a lamellar phase lipid bilayer encapsulating an aqueous core.

Lysophosphatidylcholines (LPC, lysoPC, Formula I), also called lysolecithins, are a class of chemical compounds which are derived from phosphatidylcholines. They result from partial hydrolysis of phosphatidylcholines, which removes one of the fatty acid groups.

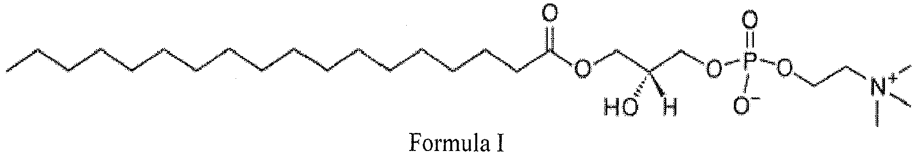

A lysophosphatidylglycerol (LPG, lyso PG, Formula II) is a type of chemical compound derived from a phosphatidyl glycerol, which is typical of cell membranes. Lyso PG results from partial hydrolysis of phosphatidyl glycerol, which removes one of the fatty acid groups.

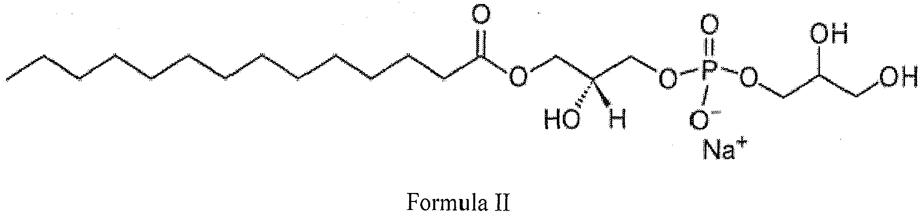

The term 'stable' as used herein means that the lipid related degradation impurities are within acceptable limits so that the lamellar structure of the liposomes is retained and micellar structures are not formed. Without wishing to be bound by any theory, inventors believe that formation of micellar structures may lead to alteration in the pharmacokinetic behavior when the liposomes are administered parenterally. In one preferred aspect, the liposomal composition is said to be stable when the lyso-phosphatidylcholine impurity remains between 0.1 to 3.0 % by weight of the phosphatidylcholine and lyso phosphatidyl glycerol impurity remains between 0.1 to 4.0 % by weight of the phosphatidylglycerol.

The lipidic impurities i.e. Lysophosphatidylcholines and lysophosphatidylglycerol may be estimated using high performance liquid chromatography. The lipidic impurities, lysophosphatidylcholines and lysophosphatidylglycerol, are expressed as % by weight of the initial amount of respective lipids. For instance, lysophosphatidylcholine is reported as % by weight of the phosphatidyl choline (such as HSPC). And lysophosphatidylglycerol is expressed as % by weight of the phosphatidyl glycerol (such as DSPG). Alternatively, the lipidic impurities may be reported as milligram per vial, i.e. the amount of impurities, in milligram, present in a vial having 50 mg of polyene antibiotic in 12.5 ml of liposomal suspension (in buffer). In preferred embodiments, the liposomal composition prepared by the process of the present invention have lyso-phosphatidylcholine impurity between 0.1 to 3.0 % by weight of the phosphatidylcholine and lyso phosphatidyl glycerol impurity between 0.1 to 4.0 % by weight of the phosphatidylglycerol.

The disclosure of United States patent no. 5,965,156 is incorporated herein by reference. The '156 patent had described a process of formation of a unilamellar liposomal composition containing a polyene antibiotic. The '156 patent describes a process for solubilizing a polyene antibiotic, comprising forming a soluble complex between the polyene antibiotic and a phosphatidylglycerol in an acidified organic solvent. It further describes a process for the formation of liposomes containing a polyene antibiotic, comprising forming a soluble complex between a polyene antibiotic and a phosphatidylglycerol in an acidified organic solvent and maintaining said soluble complex at a pH of 4.5 or less, removing the organic solvent to form a dried complex, and hydrating the dried complex with an aqueous buffer solution to form liposomes having a diameter of less than 2.0 µm.

The present invention provides a process for the preparation of an unilamellar liposomal composition containing a polyene antibiotic, said process comprising forming a soluble complex between the polyene antibiotic and a phospholipid in an acidified organic solvent and maintaining said soluble complex at a pH of 4.5 or less, removing the organic solvent to form a dried complex and hydrating the dried complex with an aqueous buffer solution and forming a dispersion of unilamellar liposomes containing the polyene antibiotic from the hydrated mixture, subjecting the dispersion to filtration sterilization,
the process comprising additional step of adding a buffer having a pH of 3 to 6 to the dispersion of unilamellar liposomes before the step of filtration sterilization.

The first step of the process of the present invention involves formation of a soluble complex between the polyene antibiotic and a phospholipid. For this, a phospholipid is dissolved in an organic solvent followed by adjusting the pH of the solution in the range of pH 1.0 to 3.0, using an acid such as hydrochloric acid. The lipid dissolution in organic solvent may be effected by warming to a temperature of about 60-70°C. Subsequently, a specified amount of polyene antibiotic drug previously dispersed in an organic solvent is added to the acidic lipid solution as obtained before, under stirring at a temperature of about 25°C - 45°C, to get a clear solution. The pH of the said soluble complex is subsequently maintained at a pH of 4.5 or less. In this step, in one embodiment, the phospholipid is phosphatidyl glycerol. However, it is possible to use any other phospholipid. In one preferred embodiment, wherein the process involves use of an organic solvent comprising chloroform and methanol in a volume ratio ranging from about 65:35 to 85:15, the inventors found that the step of formation of drug-lipid complex could be carried out at a temperature of 45°C or below and the process result in lesser degradation of drug, such that the increase in the drug related total impurities were much lower as compared to the levels obtained when a process similar to prior art process was followed which uses a mixture of chloroform and methanol in a volume ratio of 1:1, involves heating of the drug-lipid mixture to a temperature of 65°C at the time of complex formation and do not involve the additional step of adding a buffer having a pH of 3 to 6 to the dispersion of unilamellar liposomes before the step of filtration sterilization.

In preferred embodiments, the step of formation of a soluble complex between the polyene antibiotic and a phospholipid particularly comprise forming a soluble complex first between a phosphatidylglycerol (a phospholipid) and a polyene antibiotic drug as described above, along with an additional step of adding other phospholipids like phosphatidylcholine and steroidal lipids such as cholesterol or its derivatives to the complex. These additional lipids are dissolved in an organic solvent by warming at 40°C-70°C, to get a clear solution and then added to the clear solution of drug-lipid complex. Once the solution of these lipids is added to the phosphatidylglycerol-drug complex, the pH of the final solution is adjusted to about 3.0 to 4.0 and maintained at a pH of 4.5 or less. It is possible to add other excipients like antioxidants for eg. alpha-tocopherol to the above solution of drug-lipid complex.

The next step according to the process of the present invention includes, removing the organic solvent from the drug-lipid complex solution to form a dried complex. The step of removing the organic solvent from the drug-lipid complex solution to form a dried complex may be performed by known techniques like spray drying in a spray dryer, rotatory evaporation in a round bottom flask leaving a dry film, drying under vacuum and the like, preferably by industrially feasible spray drying.

The dried complex is then hydrated with an aqueous buffer solution. Preferably, the step of hydrating the dried complex with an aqueous buffer solution comprises hydrating the dried complex in a buffer at a pH of 5.7 to 6.1 with stirring at a temperature of about 20°C-30°C, followed by warming and further stirring, for about 0.5 to 1.5 hours. This results in the formation of a dispersion of multilamellar liposomes having a pH of 5.7 to 6.1.

The dispersion of the multilamellar liposomes having a pH of 5.7 to 6.1 is further subjected to size reduction by appropriate techniques, to obtain unilamellar liposomes having mean particle size in the range of 50 nanometers to 200 nanometers. The step of size reduction to get unilamellar liposomes is accomplished by the application of shearing force. Typically, shearing force can be applied to the dispersion of large multilamellar liposomes using a suitable technique such as sonication or homogenization, or freezing and thawing, extrusion, dialyzing away a detergent solution from lipids, or other known methods. In preferred embodiments, the process of size reduction of the multilamellar liposome vesicles may be carried out by way of homogenization under pressure of 10 - 1000 bars. This results in formation of a dispersion having unilamellar liposomes. The mean particle size of unilamellar liposomes vary from about 50 nanometers to 200 nanometers (nms), preferably 60 nms to 120 nms.

The process according to the present invention involves the additional step of adding a buffer having a pH of 3 to 6 to the dispersion of unilamellar liposomes obtained after size reduction step. The buffer that may be used in this additional step may be selected from succinate buffer (mixture of sucrose and sodium succinate dibasic hexahydrate), phosphate buffer, TRIS phosphate buffered saline (pH 7.4) or tris buffered saline (pH 7.4). Preferably, the buffer is succinate buffer, acidified to a pH of 3.0 to 6.0 with hydrochloric acid. It was particularly found that this step, particularly yields liposomes that are stable, that is, the lipid related impurities are found to be in acceptable limits, particularly, lyso phosphatidylglycerol, being less than 4.0 %, i.e. between 0.1 % to 4.0 % by weight of phosphatidylglycerol; and lyso phosphatidyl choline being less than 3.0 %, i.e. between 0.1 % and 3.0 % by weight of the phosphatidyl choline. This step leads to formation of a liposomal composition having better stability and purity profile. This was particularly concluded because when the similar process was followed except the additional step of adding a buffer having a pH of 3 to 6 to the dispersion of unilamellar liposomes obtained after size reduction step, an unstable liposome of amphotericin B was obtained. This is described in details in the comparative Example 1. The resulting liposomal composition has lyso phosphatidylglycerol content of 8.33 % by weight of phosphatidylglycerol and lyso phosphatidylcholine content of 5.07 % by weight of phosphatidylcholine.

The process further comprises a step of subjecting the dispersion of unilamellar liposomes to filtration sterilization. The filtration sterilization may be carried out by using a 0.22 micron filter. This results in formation of sterile liposomal composition having dispersion of unilamellar liposomes.

The process of the present invention optionally comprise a further step of lyophilisation wherein the filtered liposomal dispersion is filled in a suitable container such as a vial and subjected to lyophilization under suitable conditions in a lyophilizer, to obtain lyophilized liposomal composition in the form of a dried cake or plug of material. In one aspect, the present invention provides a lyophilized liposomal composition of a polyene antibiotic drug prepared by the process as described herein.

The present invention in one aspect or embodiment provides a process of preparing a liposomal composition, said process comprising the steps of:
a) dissolving specified amount of a phospholipid selected from the group consisting of phosphatidyl glycerol in an organic solvent, and adjusting the pH of solution in the range of pH 1.0 to 3.0,
b) adding specified amount of a polyene antibiotic drug to an organic solvent to obtain a dispersion,
c) adding the dispersion of step b), to the solution of step a), to get a clear solution,
d) dissolving at least a steroidal lipid and a phospholipid selected from the group consisting of phosphatidyl choline in an organic solvent and adding to the solution of step c),
e) drying the resultant solution of step d) to obtain a dry complex,
f) hydrating the dried complex of step e) in a buffer to obtain a dispersion of multilamellar liposomes,
g) subjecting the dispersion of step f) to size reduction to obtain a dispersion of unilamellar liposomes having a mean particle size in the range of 50 nanometers to 200 nanometers,
h) adding a buffer having a pH of 3.0 to 6.0 to the dispersion of step g),
i) subjecting the dispersion to filtration sterilization, and optionally subjecting the filtered liposomal dispersion to lyophilization to obtain lyophilized liposomal composition.

In one preferred embodiment, the present invention provides a process of preparing a liposomal composition, said process comprising the steps of:
a) dissolving specified amount of distearoyl phosphatidyl glycerol, in an organic solvent comprising chloroform and methanol in a volume ratio ranging from about 65:35 to 85:15, and adjusting the pH of solution in the range of pH 1.0 to 3.0,
b) adding specified amount of a amphotericin B drug to an organic solvent comprising chloroform and methanol in a volume ratio ranging from about 65:35 to 85:15, to obtain a dispersion,
c) adding the dispersion of step b), to the solution of step a), and mixing at a temperature below 45°C to get a clear solution,
d) dissolving at least a steroidal lipid selected from cholesterol and a phospholipid selected form hydrogenated soya phosphatidyl choline, in an organic solvent comprising chloroform and methanol in a volume ratio ranging from about 65:35 to 85:15 and adding to the solution of step c),
e) drying the resultant solution of step d) to obtain a dried complex,
f) hydrating the dried complex of step e) in a buffer at a pH of 5.7 to 6.1, to obtain a dispersion of multilamellar liposomes,
g) subjecting the dispersion obtained in step f) to size reduction such as by way of homogenization under pressure to obtain a dispersion of unilamellar liposomes having a mean particle size in the range of 50 nanometers to 200 nanometers,
h) adding a buffer having a pH of 3.0 to 6.0 to the dispersion of step g),
i) subjecting the dispersion to filtration sterilization, and
j) subjecting the filtered liposomal dispersion to lyophilization to obtain lyophilized liposomal composition.

The present invention in another embodiment provides a liposomal composition obtained by a process comprising the steps of:
a) dissolving specified amount of a phospholipid selected from the group consisting of phosphatidyl glycerol in an organic solvent, and adjusting the pH of solution in the range of pH 1.0 to 3.0,
b) adding specified amount of a polyene antibiotic drug to an organic solvent to obtain a dispersion,
c) adding the dispersion of step b), to the solution of step a), to get a clear solution,
d) dissolving at least a steroidal lipid and a phospholipid selected from the group consisting of phosphatidyl choline in an organic solvent and adding to the solution of step c),
e) drying the resultant solution of step d) to obtain a dry complex,
f) hydrating the dried complex of step e) in a buffer to obtain a dispersion of multilamellar liposomes,
g) subjecting the dispersion of step f) to size reduction to obtain a dispersion of unilamellar liposomes having a mean particle size in the range of 50 nanometers to 200 nanometers,
h) adding a buffer having a pH of 3.0 to 6.0 to the dispersion of step g),
i) subjecting the dispersion to filtration sterilization, and optionally subjecting the filtered liposomal dispersion to lyophilization to obtain lyophilized liposomal composition.

The polyene antibiotic that can be incorporated according to the process of the present invention includes, but is not limited to, amphotericin B, nystatin, natamycin, perimicin, rimocidin or candicin or the like. According to one embodiment, the polyene antibiotic is Amphotericin B. Amphotericin B may be present in the liposomal composition at a concentration of about 0.1 mg/ml to 10 mg/ml, such as for example, 0.2, 0.3, 0.4, 0.5, 0.6, 0.7, 0.8, 1.0, 2.0, 3.0, 4.0, 5.0, 6.0, 7.0, 8.0 or 9.0 mg/ml, preferably, from about 1, 2, 3, 4, 5, 6, 7 or 8 mg/ml.

The phospholipids that may be used according to the process of the present invention, preferably includes phosphatidylglycerols selected from the group consisting of, but not limited to, distearoylphosphatidylglycerol, dilaurylphosphatidylglycerol and dimyristoylphosphatidyl glycerol or salts thereof. In one preferred embodiment, distearoylphosphatidylglycerol sodium salt (DSPG-Na) is used. The additional phospholipids that may be used include phosphatidylcholines such as hydrogenated egg phosphatidycholine, hydrogenated soya phosphatidycholine, distearoyl phosphatidylcholine or dipalmitoyl phosphatidylcholine and the like. In one preferred embodiment, hydrogenated soya phosphatidycholine is used. The steroidal lipid that may be used include cholesterol or its derivatives or other sterols, such as ergosterol, stigmosterol, or androsterone. In one preferred embodiment, cholesterol is used.

The organic solvent that may be used according to the process of the present invention include, but is not limited to, chloroform, methanol, ethanol, isopropyl alcohol, ether, dichloromethane, dimethylsulfoxide, dimethylformamide and the like and mixtures thereof. Preferably, the organic solvent comprises a mixture of chloroform and methanol which may be used in a volume ratio ranging from about 15:85 to 85:15. In preferred embodiments, the organic solvent comprises mixture of chloroform and methanol in a volume ratio ranging from about 65:35 to 85:15. In one preferred embodiment, the organic solvent comprises a mixture of chloroform and methanol in a volume ratio of 75:25.

The buffer that may be used according to the present invention may be selected from succinate buffer (a mixture of sucrose and sodium succinate dibasic hexahydrate), phosphate buffer, TRIS phosphate buffered saline (pH 7.4), tris buffered saline (pH 7.4) and the like. Preferably, the buffer is succinate buffer. The buffer may be either acidified with an acid like hydrochloric acid or alkalized with an alkali like sodium hydroxide, to produce a solution having desired pH.

The present invention in one aspect provides a liposomal composition comprising:
amphotericin B in an amount ranging from 1% to 8 % by weight,
phosphatidylglycerol in an amount ranging from 2 % to 10 % by weight, phosphatidylcholine in an amount ranging from 10 % to 20 % by weight, and cholesterol in an amount ranging from 2 % to 8 % by weight of final liposomal composition,
wherein the lyso phosphatidylcholine impurity is between 0.1 to 3.0 % by weight of the phosphatidylcholine and lyso phosphatidylglycerol impurity is between 0.1 to 4.0 % by weight of the phosphatidylglycerol, when tested at any time during the shelf life of the product, when the liposomal composition is kept at either 25°C / 60 % relative humidity or 40°C / 75 % relative humidity.

The present invention in one aspect or embodiment provides a liposomal composition comprising:
amphotericin B in an amount ranging from 1 % to 8 % by weight,
phosphatidylglycerol in an amount ranging from 2 % to 10 % by weight, phosphatidylcholine in an amount ranging from 10 % to 20 % by weight, and
cholesterol in an amount ranging from 2 % to 8 % by weight of final liposomal composition, wherein the liposomal composition is obtained by a process comprising forming a soluble complex between the polyene antibiotic and a phospholipid in an acidified organic solvent and maintaining said soluble complex at a pH of 4.5 or less, removing the organic solvent to form a dried complex and hydrating the dried complex with an aqueous buffer solution and forming a dispersion of unilamellar liposomes containing the polyene antibiotic from the hydrated mixture, subjecting the dispersion to filtration sterilization, the process comprising additional step of adding a buffer having a pH of 3 to 6 to the dispersion of unilamellar liposomes before the step of filtration sterilization,
wherein the lyso phosphatidylcholine impurity is between 0.1 to 3.0 % by weight of the phosphatidylcholine and lyso phosphatidylglycerol impurity is between 0.1 to 4.0 % by weight of the phosphatidylglycerol, when tested at any time during the shelf life of the product, when the liposomal composition is kept at either 25°C / 60 % relative humidity or 40°C / 75 % relative humidity.

In one or more embodiments, the final liposomal composition comprises amphotericin B in an amount ranging from 1% to 8 % by weight, such as for example 1.5, 2.0, 2.5, 3.0, 3.5, 4.0, 4.5, 5.0, 5.5, 6.0, 6.6, 7.0, 7.5 % or intermediate ranges thereof; phosphatidylglycerol in an amount ranging from 3 % to 10 % by weight, such as for example 3.5, 4.0, 4.5, 5.0, 5.5, 6.0, 6.6, 7.0, 7.5, 8.0, 8.5, 9.0, 9.5 % or intermediate ranges thereof; phosphatidylcholine in an amount ranging from 10 % to 20 % by weight, such as for example 10.5, 11.0, 11.5, 12.0, 12.5, 13.0, 13.5, 14.0, 14.5, 15.0, 15.5, 16.0, 16.6, 17.0, 17.5, 18.0, 18.5, 19.0, 19.5 or intermediate ranges thereof; cholesterol in an amount ranging from 2 % to 8 % by weight, such as for example 2.5, 3.0, 3.5, 4.0, 4.5, 5.0, 5.5, 6.0, 6.6, 7.0, 7.5 % or intermediate ranges thereof; expressed % by weight of final liposomal composition, which further includes buffer comprising sucrose and sodium succinate and antioxidant such as alpha-tocopherol.

The present invention in one preferred embodiment provides a liposomal composition comprising:
Amphotericin B in an amount of about 3.5 % by weight,
phosphatidyl glycerol in an amount of about 6.0 % by weight,
phosphatidylcholine in an amount of about 15.0 % by weight, and
cholesterol in an amount of about 3.5 % by weight of final liposomal composition,
wherein the liposomal composition is obtained by a process comprising forming a soluble complex between the polyene antibiotic and a phospholipid in an acidified organic solvent and maintaining said soluble complex at a pH of 4.5 or less, removing the organic solvent to form a dried complex and hydrating the dried complex with an aqueous buffer solution and forming a dispersion of unilamellar liposomes containing the polyene antibiotic from the hydrated mixture, subjecting the dispersion to filtration sterilization, the process further comprise additional step of adding a buffer having a pH of 3 to 6 to the dispersion of unilamellar liposomes before the step of filtration sterilization,
wherein the lyso phosphatidylcholine impurity is about 1.83 % by weight of the phosphatidylcholine and lyso phosphatidyl glycerol impurity is about 2.98 % by weight of the phosphatidylglycerol.

In contrast, when a prior art liposome composition is tested, it was found to yield higher levels of lipid impurities. For example when Ambisome^{®} is tested, it was found to have lyso phosphatidylcholine impurity of about 3.38 % by weight of the phosphatidylcholine and lyso phosphatidyl glycerol impurity of about 4.40 % by weight of the phosphatidylglycerol, when tested during the shelf life of the product, when the liposomal composition is kept at room temperature (25°C / 60 % relative humidity). And when the liposomal composition prepared according to the prior art process as given in comparative example 2 was tested, it was found to have lyso phosphatidylcholine impurity of about 5.82 % by weight of the phosphatidylcholine and lyso phosphatidyl glycerol impurity of about 7.86 % by weight of the phosphatidylglycerol. The process of the present invention provides stabilization of the lipids, the drug used, and the structure of vesicles so formed. The liposome vesicles so formed have well defined, spherical shape and show high % drug entrapment, optimum therapeutic efficacy and low toxicity. In one or more preferred embodiments, high percentage of Amphotericin B of the order of more than 99.5% gets entrapped into the liposome. The process is capable of reproducible continuous flow production with commercial feasibility.

The liposomal composition of the present invention was subjected to in-vitro toxicity study by potassium release test using rat red blood cells (RBCs) and compared with liposomes prepared according to the prior art processes. The K₅₀ values were determined. K₅₀ value refers to the concentration of drug in µg/ml at which 50% of potassium gets released from the RBCs. The K₅₀ value results demonstrate the comparative availability of amphotericin B to mammalian cells and intrinsic toxicity of liposomal compositions. It was observed that liposomal composition of the present invention show much reduced intrinsic toxicity to mammalian cells compared to the liposomal compositions of the prior art, prepared as per prior art processes.

The liposomal composition of the present invention was subjected to long term stability studies at varying storage conditions i.e. at room temperature (25°C/60% relative humidity) and accelerated stability conditions (temperature of 40°C/75% relative humidity). It was observed that the liposomes prepared by the process of the present invention remains stable, physically and chemically, upon storage at room temperature, for prolonged period of time, such as at least 6 months, preferably 1 year. The liposomes remains physically stable, such that the morphology and size of the liposomes does not change upon storage i.e. the liposomes even when tested after storage show well defined spherical shape and there occurs negligible change in the mean particle size. The liposomes remain chemically stable upon storage, such that the drug content and the content of total impurities do not rise significantly, upon storage.

In the context of this specification "comprising" is to be interpreted as "including". Where technically appropriate, embodiments of the invention may be combined. Embodiments are described herein as comprising certain features/elements. The disclosure also extends to separate embodiments consisting or consisting essentially of said features/elements. Any embodiments specifically and explicitly recited herein may form the basis of a disclaimer either alone or in combination with one or more further embodiments.

Hereinafter, embodiments of the present disclosure will be more specifically described by way of examples. The examples should not be understood as limiting the scope of the present disclosure, and are merely used as illustrations of specific embodiments of the present disclosure.

### EXAMPLE 1

This example illustrates a specific embodiment of the present invention, and provides for the process of preparing liposomal composition comprising the drug Amphotericin B:
Chloroform and methanol were mixed in a volume ratio of 75:25 to form a solvent mixture which was used in further processing steps. Specified amounts of distearoyl phosphatidyl glycerol sodium salt (eq. to 84 mg/vial DSPG) was dissolved in chloroform and methanol (75:25) solvent mixture, and warming at a temperature of about 60-70°C. To this solution, hydrochloric acid was added to attain a final pH in the range of 1.0 to 3.0. In a separate container, specified amount of Amphotericin B (50 mg/vial) was dispersed in chloroform-methanol (75:25) solvent mixture. This Amphotericin B dispersion was added to acidified distearoyl phosphatidyl glycerol sodium salt solution with stirring at 25-40°C. This resulted in a translucent orange coloured solution comprising Amphotericin B- distearoyl phosphatidyl glycerol sodium salt lipophilic complex. In separate containers, specified amounts of hydrogenated soya phosphatidyl choline (213 mg/vial) and cholesterol (52 mg/vial) were dissolved in chloroform and methanol solvent mixture at a temperature of 40-70°C to get a clear solution. The hydrogenated soya phosphatidyl choline solution and cholesterol solution were then added and mixed with Amphotericin B- distearoyl phosphatidyl glycerol sodium solution. The pH of the final solution was adjusted to about 3.0-4.0. Alpha tocopherol (0.64 mg/vial) pre-dissolved in methanol was added to the above solution with stirring. The solution of Amphotericin B- distearoyl phosphatidyl glycerol sodium and other lipids so obtained were spray dried to remove the organic solvents and get the free flowing powder. The powder of the drug lipid complex was analyzed for drug related total impurities. The powder so formed was hydrated in a buffer at a pH of 5.7 to 6.1 with stirring at controlled room temperature followed by warming and further stirring for 0.5 to 1.5 hrs. The hydrated lipidic dispersion having multilamellar vesicles was subjected to homogenization under pressure, which resulted in formation of lipidic dispersion having unilamellar vesicles with a mean particle size in the range of 50-150 nms. After completion of size reduction, the 'sucrose - sodium succinate dibasic hexahydrate' buffer system acidified to a pH of 3.0 to 6.0 was added to the dispersion with stirring. The bulk was sterilized by filtering through a series of sterile filters. Subsequent to this, a measured volume of the filtered bulk dispersion was aseptically filled in vial and lyophilized. The percentage drug entrapment was determined and it was found to be 99.91% of the label claim.

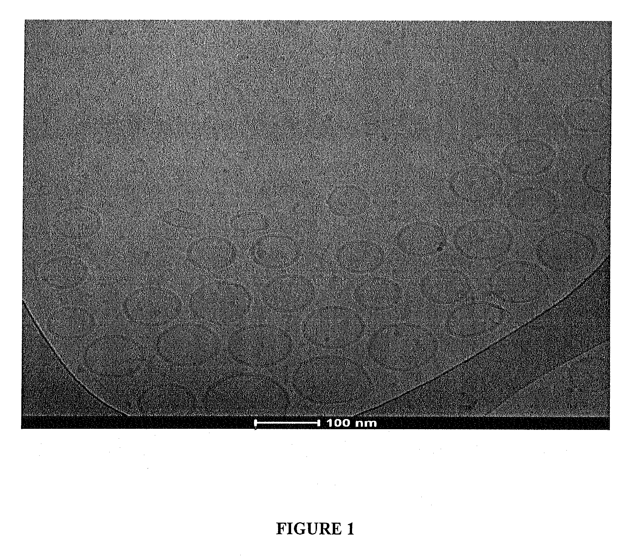

Morphological examination: The liposomal composition was examined for its structures by cryo-transmission electron microscopy and the microscopic image is provided in Figure 1. The image show that the liposomal vesicles are structured, spherical and uniform in shape.

### COMPARATIVE EXAMPLE 1

Amphotericin B liposomal composition was prepared following a method similar to the one described in Example 1, except that the process did not involved the step of addition of buffer after the formation of unilamellar liposomes, ie. No buffer was added after the size reduction or homogenization step.

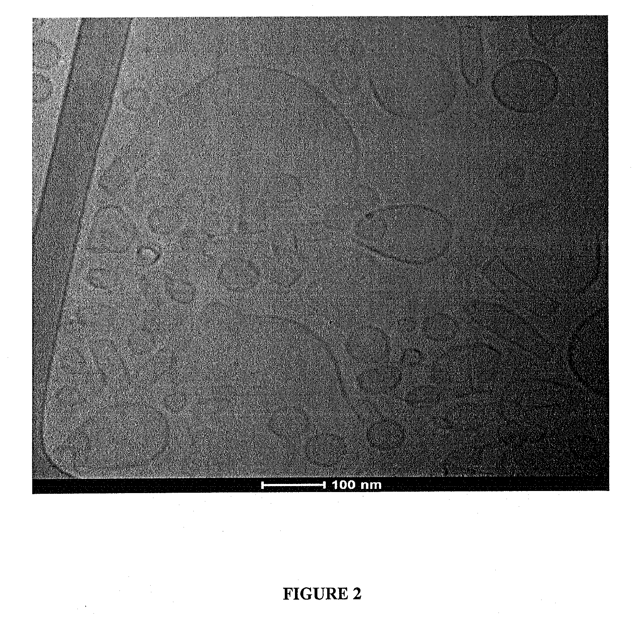

Morphological examination: The liposomal composition prepared as per comparative example 1 was examined for its structures by cryo-transmission electron microscopy and the microscopic image is provided in Figure 2. The Figure 2 shows that the liposomal vesicles have distorted, uneven vesicular structures.

### COMPARATIVE EXAMPLE 2

This example illustrates preparation of Amphotericin B liposomes following a prior art known method. Particularly, a process similar to one described in US patent number 5,965,156 was followed.

Chloroform and methanol were mixed in a volume ratio of 50:50 to form a solvent mixture which was used in further processing steps. Specified amounts of distearoyl phosphatidyl glycerol sodium salt (eq. to 84 mg/vial DSPG) was dissolved in chloroform and methanol (50:50) solvent mixture, and warming at a temperature of about 60-70°C. To this solution, hydrochloric acid was added to attain a final pH in the range of 1.0 to 3.0. In a separate container, specified amount of Amphotericin B (50 mg/vial) was dispersed in chloroform-methanol (50:50) solvent mixture. This Amphotericin B dispersion was added to acidified distearoyl phosphatidyl glycerol sodium solution with stirring at 65°C. This resulted in a translucent orange coloured solution comprising Amphotericin B- distearoyl phosphatidyl glycerol sodium lipophilic complex. In separate containers, specified amounts of hydrogenated soya phosphatidyl choline (213 mg/vial) and cholesterol (52 mg/vial) were dissolved in chloroform and methanol solvent mixture at a temperature of 60-70°C to get a clear solution. The hydrogenated soya phosphatidyl choline solution and cholesterol solution were then added and mixed with Amphotericin B- distearoyl phosphatidyl glycerol sodium solution. The pH of the final solution was adjusted to about 3.0-4.5. Alpha tocopherol (0.64 mg/vial) pre-dissolved in methanol was added to the above solution with stirring. The solution of Amphotericin B-distearoyl phosphatidyl glycerol sodium and other lipids so obtained were spray dried to remove the organic solvents and get the free flowing powder. The powder of the drug lipid complex was analyzed for drug related total impurities. The powder so formed was hydrated in a buffer at a pH of 5.5 with stirring and warming for 40-60 minutes. The hydrated lipidic dispersion having multilamellar vesicles was subjected to homogenization, which resulted in formation of lipidic dispersion having unilamellar vesicles. The bulk was sterilized by filtering through a series of sterile filters. Subsequent to this, a measured volume of the filtered bulk dispersion was aseptically filled in vial and lyophilized.

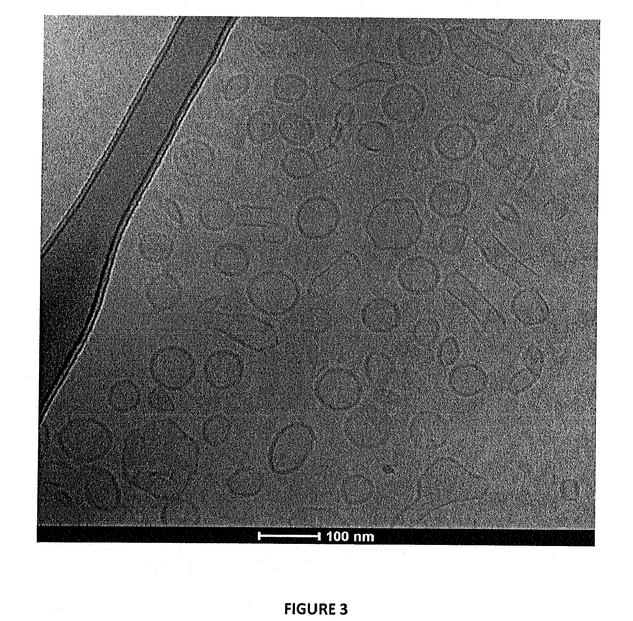

Morphological examination: The liposomal composition prepared as per comparative example 2 was examined for its structures by cryo-transmission electron microscopy and the microscopic image (figure 3) showed that the liposomal vesicles have distorted uneven vesicular structures.

### EXAMPLE 2

Estimation of Lipidic Impurities: The liposomal compositions of Example 1, comparative example 1 and comparative example 2, were subjected to estimation of lipidic impurities such as Lyso-Phosphatidyl glycerol and Lyso-Phosphatidyl choline. The lipid impurities were determined by liquid chromatographic technique, viz. HPLC. The HPLC method utilized a C18 chromatographic column, with mobile phase being a mixture of acetate buffer, methanol, tetrahydrofuran and triethylamine and the diluent being a mixture of methanol and chloroform, the detector being Refractive index at 40°C temperature. The content of lipidic impurities is given in Table 1 below.

**Table 1: Results of lipid impurity levels of the liposome composition prepared according to the present invention, and as per prior art comparative examples.**

| Liposome preparation of | Condition at which tested | Time | Lyso-Phosphatidylcholine : % by weight of Phosphatidylcholine label claim | LysoPhosphatidylglycerol : % by weight of Phosphatidylglycerol label claim |
|---|---|---|---|---|
| Example 1 | 25°C/60% RH | Initial | 1.45 | 3.09 |
| | 25°C/60% RH | 6 months | 1.83 | 2.98 |
| | 40°C/75% RH | 6 months | 1.83 | 2.98 |
| Ambisome^{®} | 25°C/60% RH | 6 months | 2.72 | 3.69 |
| | 25°C/60% RH | 12 month | 3.38 | 4.40 |
| Comparative example 1 | 25°C/60% RH | Initial | 5.07 | 8.33 |
| Comparative example 2 | 25°C/60% RH | Initial | 5.82 | 7.86 |

| | | | | |
|---|---|---|---|---|
| RH- Relative Humidity. Ambisome^{®}: Liposomal preparation developed by Vestar Inc. U.S.A. approved by USFDA | | | | |

Based on all of the above results of either the physical or chemical characterization of the liposome compositions obtained by the process of Example 1, Ambisome^{®} marketed product, comparative example 1 and comparative example 2, it has been found that - the liposomal composition of Example 1 had very intact uniform, lamellar structure (see Figure 1) as against the liposomal composition prepared according to comparative examples 1 and 2, (see Figure 2 & 3 respectively) which show distorted morphology with uneven size and shape. Lipid analysis results described in Table 1 showed that the liposome composition prepared according to Example 1, had very low levels of lipid impurities, suggesting the composition to be stable after preparation and storage over a period of extended duration. On the other hand, the liposome compositions prepared as per prior art processes, in fact yielded unstable liposome compositions in that the hydrolysis impurities of the phospholipids, immediately upon preparations were itself high, and therefore, did not require further investigation for its further increase. The above results established that the present invention provides a liposomal composition that is stable, and better than the prior known/comparative compositions.

## Claims

1. A process for the preparation of an unilamellar liposomal composition containing a polyene antibiotic, said process comprising forming a soluble complex between the polyene antibiotic and a phospholipid in an acidified organic solvent and maintaining said soluble complex at a pH of 4.5 or less, removing the organic solvent to form a dried complex and hydrating the dried complex with an aqueous buffer solution and forming a dispersion of unilamellar liposomes containing the polyene antibiotic from the hydrated mixture, subjecting the dispersion to filtration sterilization,
the process comprising additional step of adding a buffer having a pH of 3 to 6 to the dispersion of unilamellar liposomes before the step of filtration sterilization.

2. The process as claimed in claim 1, wherein the liposomal composition contains lyso phophatidylglycerol at a concentration ranging from 0.1 % to 4.0 % by weight of phosphatidylglycerol and lyso phosphatidylcholine at a concentration ranging from 0.1% to 3.0 % by weight of phosphatidylcholine.

3. The process as claimed in claim 1, wherein said polyene antibiotic is amphotericin B.

4. The process as claimed in claim 1, wherein the step of hydrating the dried complex with an aqueous buffer solution comprises hydrating the dried complex in a buffer at a pH of 5.7 to 6.1 along with stirring at a temperature of about 20-30°C, followed by warming and further stirring.

5. The process as claimed in claim 1, wherein the buffer in the additional step comprises sodium succinate and sucrose, acidified to a pH of 3.0 to 6.0.

6. A liposomal composition obtained by the process as claimed in claim 1.

7. The process as claimed in claim 1, wherein the process further comprises filling the sterilized dispersion into a container and subjecting it to lyophilisation to obtain a lyophilized composition.

8. A lyophilized liposomal composition obtained by the process as claimed in claim 7.
